# FASCICULE DE BREVET EUROPEEN

(11) **EP 2 928 541 B1**
(45) Date de publication et mention de la délivrance du brevet: **28.08.2019**
(21) Numéro de dépôt: 13802930.1
(22) Date de dépôt: 05.12.2013
(51) Int. Cl.: A61M 39/22, A61M 39/10

(54) **ENSEMBLE À USAGE MÉDICAL POUR L'ADMINISTRATION D'UN PRODUIT À UN PATIENT**
ANORDNUNG ZUR MEDIZINISCHEN VERWENDUNG ZUR VERABREICHUNG EINES PRODUKTS AN EINEN PATIENTEN
ASSEMBLY FOR MEDICAL USE FOR ADMINISTERING A PRODUCT TO A PATIENT

(30) Priorité: 05.12.2012 FR 1261699
(43) Date de publication de la demande: 14.10.2015
(73) Titulaire: Guerbet, 93420 Villepinte (FR)
(72) Inventeur: BRAULT-GUYON, Nathanaëlle, F-86350 Saint Secondin (FR); HANNIGSBERG, Morgane, F-73300 Saint Jean De Maurienne (FR); GUERDER, Philippe, F-95400 Villiers Le Bel (FR)
(74) Mandataire: Regimbeau
(86) Numéro de dépôt international: PCT/EP2013/075615
(87) Numéro de publication internationale: WO 2014/086904

(56) Documents cités:
- EP-A1- 0 028 198
- EP-A1- 0 998 952
- EP-A2- 2 550 985
- DE-A1- 2 907 832
- FR-A1- 2 475 903
- GB-A- 2 067 075
- US-A- 5 984 373

## Description

### DOMAINE TECHNIQUE

La présente invention concerne le domaine technique général de l'administration de produit, notamment liquide, à un patient, par exemple par voie parentérale, éventuellement à partir d'une poche souple.

### PRESENTATION GENERALE DE L'ART ANTERIEUR

Les ensembles à usage médical comprenant une tubulure couplée par l'une de ses extrémités à un connecteur de type Luer - femelle ou mâle - sont bien connus, et couramment utilisés pour connecter différents dispositifs médicaux, notamment des tubes conduisant un produit liquide ou visqueux - tel qu'un produit de contraste - à administrer à une personne.

Différents types d'injecteurs peuvent être utilisés pour injecter un tel produit liquide ou visqueux, comme par exemple :
- un injecteur de type « *pousse-seringue »* comprenant un logement destiné à recevoir une seringue contenant le produit à administrer, ou encore
- un injecteur de type « *à poche »* - tel que décrit dans le document EP 0 852 152 - comprenant un logement destiné à recevoir une poche souple contenant le produit à administrer.

Lorsque la viscosité du produit à administrer est importante, la pression générée dans l'ensemble à usage médical composé du connecteur et de la tubulure peut être très importante.

Cette pression importante peut induire l'apparition de fuites à la jonction entre la tubulure et le connecteur de l'ensemble à usage médical.

Le document FR 2 475 903 décrit un dispositif de connexion pour raccorder des tuyaux ou analogues. Le dispositif comprend un raccord de connexion tronconique et une pièce tubulaire entourant le raccord de connexion, la pièce tubulaire formant un moyen de préhension permettant de faciliter l'enfilage d'un tuyau souple sur le raccord de connexion.

Le document EP 0 028 198 décrit un dispositif de connexion incluant un embout mâle et un embout femelle destinés à coopérer ensembles. L'embout femelle comporte une jupe destinée à coopérer soit avec une jupe d'un bouchon, soit avec une jupe de l'embout mâle.

DE 29 07 832 décrit un ensemble à usage médical ayant les caractéristiques du préambule de la revendication 1.

Un but de la présente invention est de proposer un ensemble à usage médical permettant de pallier l'inconvénient précité. Plus précisément, un but de la présente invention est de proposer un ensemble à usage médical composé d'une tubulure et d'un connecteur apte à supporter des pressions supérieures à 11-12 bars, voire supérieures à 15 ou 20 bars.

### PRESENTATION DE L'INVENTION

A cet effet, l'invention propose un ensemble à usage médical pour l'administration d'un produit à un patient comprenant les caractéristiques de la revendication 1. Le fait que les dimensions de l'appendice et de la jupe du connecteur soient adaptées aux dimensions de la tubulure pour permettre l'application de contraintes sur celle-ci permet de limiter les risques de fuite à la jonction entre le connecteur et la tubulure. En effet, ceci permet à la zone d'interface d'exercer sur la tubulure des ensembles de forces radiales dans des sens opposés tendant à écraser la tubulure dans son épaisseur.

L'appendice et la jupe sont monoblocs. Le fait que la zone d'interface soit réalisée en une seule pièce permet d'améliorer l'étanchéité de l'ensemble à la jonction entre le connecteur et la tubulure. Plus précisément, le fait que l'appendice et la jupe soient solidaires limite les risques de fuite par rapport à une zone d'interface constituée de plusieurs éléments liés entre eux par soudage ou collage. En effet, dans le cas d'une injection à hautes pressions, des fuites peuvent se produire par exemple aux zones de liaison des différents éléments constituant la zone d'interface si celle-ci est constituée en plusieurs pièces.
La connexion entre la tubulure et le connecteur peut être réalisée par un emmanchement en force de la tubulure dans la gorge de réception définie entre l'appendice et la jupe du connecteur.

Avantageusement, l'extrémité du connecteur opposée à l'extrémité comportant la jupe et l'appendice peut comprendre une tête pour son raccordement à un autre connecteur. La forme de cette tête peut varier. Elle est, par exemple, adaptée pour coopérer avec la tête d'un autre connecteur. Ceci permet le raccordement de l'ensemble à usage médical selon l'invention à des systèmes existants d'acheminement de produit à administrer à un patient.

En variante, l'extrémité du connecteur opposée à l'extrémité comportant la jupe et l'appendice peut comprendre un autre appendice destiné à être inséré dans une autre tubulure, et une autre jupe entourant l'autre appendice, les dimensions de l'autre appendice étant prévues de sorte à exercer un ensemble de forces tendant à dilater l'autre tubulure, et les dimensions de l'autre jupe étant prévues de sorte à exercer un ensemble de forces tendant à comprimer l'autre tubulure.

Le connecteur de l'ensemble à usage médical peut également comprendre un canal axial sensiblement cylindrique entre ses deux extrémités. En variante, le canal peut être coudé en fonction de l'application visée.

Par canal axial « sensiblement cylindrique », on entend un canal axial dont la base est ovale, ronde ou tronconique.

Selon l'invention, la jupe comprend une face intérieure tronconique en regard de l'appendice, le diamètre de la face intérieure de la jupe augmentant en direction de son extrémité libre. Ceci permet de faciliter l'insertion de la tubulure dans la gorge de réception. En variante, la face intérieure de la jupe peut comprendre au moins un bourrelet s'étendant vers l'appendice et dont les dimensions sont prévues pour induire l'application d'un ensemble de forces sur la tubulure, cet ensemble de forces tendant à comprimer la tubulure une fois celle-ci raccordée au connecteur.

De même, l'appendice peut comprendre une face extérieure tronconique en regard de la jupe, le diamètre de la face extérieure de l'appendice diminuant en direction de son extrémité libre. Ceci permet également de faciliter l'insertion de la tubulure dans la gorge. En variante, la face extérieure de l'appendice peut comprendre au moins un bourrelet s'étendant vers la jupe et dont les dimensions sont prévues pour induire l'application d'un ensemble de forces sur la tubulure, cet ensemble de forces tendant à dilater la tubulure une fois celle-ci raccordée au connecteur.

Outre les avantages cités ci-dessus, la combinaison :
- d'une face intérieure de jupe tronconique dont le diamètre augmente en direction de l'extrémité du connecteur en regard de la tubulure, et
- d'une face extérieure d'appendice tronconique dont le diamètre diminue en direction de l'extrémité du connecteur en regard de la tubulure,
permet de faciliter l'opération de démoulage du connecteur lors de sa fabrication.

Selon l'invention, le connecteur est monobloc. Plus précisément, le connecteur peut être réalisé en une seule pièce pour limiter les risques de jeu mécanique entre différentes pièces le constituant, comme par exemple les risques de jeu mécanique entre la jupe et l'appendice si ceux-ci font partis de deux pièces distinctes. Dans des modes de réalisation ne faisant pas partie de l'invention comme revendiquée, le connecteur peut être constitué de plusieurs pièces élémentaires (par exemple une première pièce incluant un appendice et un canal et une deuxième pièce composée de la jupe) assemblées les unes aux autres pour former le connecteur.

L'appendice peut comprendre une section frangible s'étendant selon un axe longitudinal à son extrémité libre destinée à être couplée à la tubulure. Ceci permet de maintenir l'asepsie dans la tubulure préalablement à son utilisation pour l'administration du produit au patient. De préférence, la section frangible comporte au moins une ailette s'étendant vers l'extérieur perpendiculairement à l'axe longitudinal de la section frangible pour faciliter sa séparation du connecteur. Avantageusement, la forme de l'ailette peut être en portion de sphère. Ceci permet de limiter les risques de perforation de la tubulure lors de la manipulation de celle-ci pour désolidariser la section frangible du reste du connecteur.

Dans certaines variantes de réalisation, la section frangible peut comprendre quatre ailettes s'étendant vers l'extérieur perpendiculairement à l'axe longitudinal de la section frangible, lesdites ailettes étant de forme en portion de sphère. Ceci permet de limiter Dans ce cas, les ailettes peuvent être décalées d'un angle de 90° les unes par rapport aux autres de sorte à former une boule. Cette forme d'ailettes en boule permet notamment d'éviter la rétention de microbulles par la section frangible.

De préférence, le matériau constituant le connecteur est du polycarbonate. L'avantage de ce matériau est qu'il n'est pas sujet au fluage lorsqu'il est soumis à une température élevée comme au moment de la stérilisation. Il permet ainsi de maintenir un serrage de la tubulure par le connecteur. Un matériau tel que le polyéthylène par exemple, ne permet pas d'apporter cet avantage.

De préférence, la tubulure est constituée de plusieurs (au moins deux) couches de matériaux. De façon encore plus préférentielle, le matériau constituant la partie intérieure (interne) de la tubulure est une couche d'éthylène-acétate de vinyle (ou EVA). Cette couche d'EVA a comme propriété de fondre quand elle est soumise à une température élevée, comme au moment de la stérilisation. Ceci permet d'augmenter l'adhérence entre le connecteur et la tubulure une fois ceux-ci raccordés, notamment lors de la stérilisation de la tubulure et du connecteur après leur raccordement, cette opération de stérilisation induisant le soudage du connecteur à la tubulure. On évite ainsi l'utilisation d'une colle externe pouvant poser des problèmes de biocompatibilité au contact de produits pharmaceutiques, tels que des produits de contraste.

Le matériau constituant la jupe peut être transparent ou translucide. Ceci permet de vérifier visuellement que la tubulure est insérée jusqu'au fond de la gorge de réception lors du couplage du connecteur à la tubulure. La face extérieure de la jupe peut également comprendre des cannelures pour faciliter sa manipulation, notamment lors de l'opération de raccordement du connecteur à la tubulure.

Enfin, l'ensemble à usage médical peut également comprendre une poche souple contenant le produit à administrer, cette poche étant connectée à l'autre extrémité de la tubulure.

La divulgation concerne également un connecteur à usage médical destiné à être raccordé à une tubulure, le connecteur comprenant :
- un appendice destiné à être inséré dans la tubulure, les dimensions de l'appendice étant adaptées pour exercer un ensemble de forces tendant à dilater la tubulure,
- une jupe entourant l'appendice de sorte que la jupe et l'appendice définissent une gorge pour la réception de la tubulure, les dimensions de la jupe étant adaptées pour exercer un ensemble de forces tendant à comprimer la tubulure.

L'invention concerne également l'utilisation d'un connecteur à usage médical comprenant les caractéristiques de la revendication 13.

### PRESENTATION DES FIGURES

D'autres caractéristiques et avantages de l'invention ressortiront encore de la description qui suit, laquelle est purement illustrative et non limitative et doit être lue en regard des dessins annexés, sur lesquels :
- la figure 1 illustre un exemple d'ensemble à usage médical selon l'invention,
- les figures 2 et 3 sont des vues en perspective et en coupe illustrant un exemple de connecteur de l'ensemble à usage médical.

### DESCRIPTION DE MODES DE REALISATION DE L'INVENTION

On va maintenant décrire plus en détail l'invention en référence aux figures. Dans ces différentes figures, les éléments équivalents portent les mêmes références numériques.

### Ensemble à usage médical

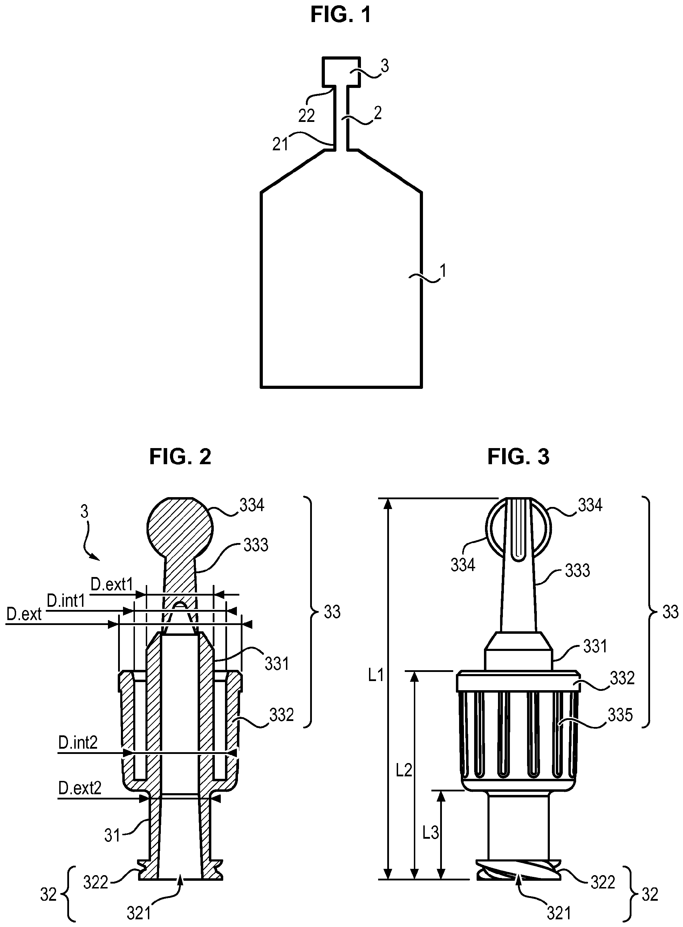

En référence à la figure 1, on a illustré un exemple d'ensemble à usage médical comprenant une poche 1, une tubulure 2 et un connecteur 3.

La poche 1 médicale comporte deux feuilles superposées de longueur et de largeur appropriées composées de plusieurs couches de films minces stratifiées en matériaux flexibles ou souples, éventuellement transparents ou translucides tels que les matériaux polymères comprenant le polyéthylène, le polypropylène, et des matériaux de préférence thermoplastiques.

L'extrémité proximale 21 de la tubulure 2 est connectée à la partie supérieure de la poche 1. Cette tubulure 2 est scellée entre les feuilles superposées. L'extrémité distale 22 de la tubulure 2 est raccordée au connecteur 3 pour le couplage de la poche 1 à un set de transfert de fluide relié au patient.

### Connecteur

Le connecteur 3 est illustré aux figures 2 et 3.

Il comprend un corps 31 sensiblement cylindrique, une zone de verrouillage 32 à l'une des extrémités du corps 31 et une zone d'interface tube 33 à l'autre extrémité du corps 31.

Un passage axial est ménagé dans le corps 31 et les zones 32, 33 pour permettre l'acheminement du liquide à administrer à travers le connecteur 3.

Le connecteur 3 est de préférence réalisé en polycarbonate. La partie intérieure (ou interne) de la tubulure est de préférence une couche d'éthylène-acétate de vinyle (ou EVA). L'utilisation de tels matériaux permet d'augmenter l'adhérence entre le connecteur 3 et la tubulure 2 après leur stérilisation une fois ceux-ci assemblés pour former l'ensemble à usage médical.

### Zone de verrouillage

La zone de verrouillage 32 du connecteur 3 comporte une tête pour le couplage de l'ensemble à usage médical à un set de transfert relié au patient.

Cette tête inclut par exemple un embout 321 destiné à pénétrer dans une tête d'un autre connecteur et/ou une collerette (non représentée) destinée à contenir la tête d'un autre connecteur.

L'embout 321 et/ou la collerette peuvent comprendre des dents 322 s'étendant radialement ou un filetage pour leur encliquetage ou leur vissage à la tête de l'autre connecteur.

Un capuchon peut être prévu pour coiffer la tête du connecteur préalablement à son couplage à un autre connecteur. Ceci permet d'éviter les risques de contamination bactérienne de l'ensemble à usage médical.

De préférence, le matériau constituant le capuchon est choisi de sorte à limiter l'adhérence entre le capuchon et la tête même après leur stérilisation.

### Zone d'interface

La zone d'interface est composée :
- d'un appendice 331 destiné à être inséré dans l'extrémité de la tubulure 2 de l'ensemble à usage médical, et
- d'une jupe 332 entourant l'appendice 331 et dans laquelle l'extrémité du tube est destinée à être insérée.

### Appendice

L'appendice 331 est de forme tronconique pour permettre son emmanchement par friction dans la tubulure 2. Le diamètre externe de l'appendice 331 est adapté au diamètre intérieur de la tubulure 2.

Plus précisément :
- au niveau de son extrémité la plus éloignée du corps, le diamètre externe de l'appendice 331 est légèrement inférieure ou égale au diamètre intérieur de la tubulure 2 ; ceci permet de faciliter l'insertion de l'appendice 331 dans la tubulure 2,
- au niveau de son extrémité la plus proche du corps, le diamètre externe de l'appendice 331 est quant à lui légèrement supérieur au diamètre intérieur de la tubulure 2 ; ainsi, lorsque l'appendice 331 est enfiché dans la tubulure 2, celui-ci exerce sur la tubulure 2 un ensemble de forces tendant à dilater la tubulure 2.

Le connecteur 3 peut comprendre une section frangible 333 à l'extrémité de l'appendice 331 opposée au corps 31 du connecteur 3. La section frangible 333 comprend au moins une ailette 334 en forme de portion de sphère s'étendant radialement vers l'extérieur.

Préférentiellement, la section frangible 333 comprend quatre ailettes 334 :
- s'étendant radialement vers l'extérieur,
- en forme de portion de sphère, et
- décalées de 90° les unes par rapport aux autres de sorte à former une boule.

Cette forme de boule des ailettes 334 permet d'éviter les risques de blessure d'un utilisateur lors de la manipulation de l'ensemble à usage médical pour séparer la section frangible du connecteur. Par ailleurs, la forme de boule permet d'éviter la rétention de microbulles. La forme en boule permet enfin d'éviter les risques de perforation de la tubulure 2 lors de la manipulation de l'ensemble à usage médical pour séparer la section frangible 333 du connecteur 3.

### Jupe

La jupe 332 s'étend à la périphérie de l'appendice 331 de sorte à entourer celui-ci, la face interne de la jupe 332 faisant face à la face externe de l'appendice 331.

Les dimensions de la jupe 332 sont adaptées pour éviter tout écartement - notamment le gonflement - de la tubulure 2 lors d'une augmentation de la pression à l'intérieur de celle-ci.

La jupe 332 est de forme tronconique pour permettre l'emmanchement par friction de la tubulure 2 dans la jupe 332. Le diamètre interne de la jupe 332 est adapté au diamètre extérieur de la tubulure 2.

Plus précisément :
- au niveau de son extrémité la plus éloignée du corps et sur une distance très courte, c'est-à-dire inférieure à 10% de la profondeur de la gorge, le diamètre interne de la jupe 332 est supérieur ou égal au diamètre extérieur de la tubulure 2;
- au niveau de son extrémité la plus proche de corps, le diamètre interne de la jupe 332 est quant à lui légèrement inférieur au diamètre extérieur de la tubulure 2.

Ainsi, lorsque la tubulure 2 est emmanchée dans la jupe 332, la jupe 332 exerce sur la tubulure 2 un ensemble de forces tendant à comprimer la tubulure 2.

La face externe de la jupe 332 peut comprendre des cannelures 335 s'étendant radialement vers l'extérieur pour faciliter la préhension de celle-ci, notamment pour le vissage/dévissage d'un capuchon monté sur la zone d'interface tube préalablement à son assemblage avec la tubulure pour former l'ensemble à usage médical.

De préférence, la jupe 332 est transparente ou translucide pour permettre de contrôler visuellement la qualité du raccordement entre le connecteur 3 et la tubulure 2. Notamment, le fait que la jupe 332 soit transparente ou translucide permet de vérifier que la tubulure 2 soit correctement insérée jusqu'au fond du volume défini par l'appendice 331 et la jupe 332.

### Combinaison appendice/jupe

On aura ainsi compris que l'appendice 331 et la jupe 332 forment une gorge pour la réception de la tubulure 2.

Cette gorge présente de préférence une forme de V en section, de sorte qu'une fois insérée dans la gorge de réception, la zone d'interface tube exerce sur la tubulure 2 des ensembles de forces radiales dans des sens opposés tendant à écraser la tubulure 2 dans son épaisseur afin d'éviter tout risque de fuite du produit à administrer à l'interface entre la tubulure 2 et le connecteur 3.

Le tableau ci-dessous donne, à titre indicatif, des exemples de valeurs :
- pour le diamètre externe (D.ext) de la jupe 332, ainsi que le diamètre interne de la jupe 332 au niveau de son extrémité la plus éloignée (D.int1) du corps 31 et au niveau de son extrémité la plus proche (D.int 2) du corps 31,
- pour le diamètre externe de l'appendice 331 au niveau de son extrémité la plus éloignée (d.ext1) du corps 31 et au niveau de son extrémité la plus proche (d.ext2) du corps 31,
- la longueur totale L1 du connecteur 3,
- la longueur L2-L3 de la zone d'interface tube du connecteur 3, et
- la longueur L3 de la zone de verrouillage du connecteur 3.

| **Cotes** | **Gammes de valeurs (en mm)** |
|---|---|
| **D.ext** | 10 à 12, préférentiellement 10,5 à 10,7 (encore plus préférentiellement 10,6) |
| **d.ext1** | 6,35 à 6,45 (préférentiellement 6,40) |
| **d.ext2** | 6,45 à 6,55 (préférentiellement 6,50) |
| **D.int1** | 8,75 à 8,85 (préférentiellement 8,80) |
| **D.int2** | 8,69 à 8,79 (préférentiellement 8,74) |
| **L1** | 30 à 40 (préférentiellement 36,5) |
| **L2** | 15 à 25 (préférentiellement 20) |
| **L3** | 7 à 10 (préférentiellement 8,5) |
| **L2 - L3** | Supérieur à 9 mm (préférentiellement ≥10) |

Ces côtes permettent à l'homme du métier de fabriquer un exemple de connecteur pouvant être utilisé pour résoudre le problème technique selon l'invention.

La longueur L2-L3 définit la zone de collage entre la tubulure et le connecteur. La différence entre les diamètres D.ext et D.int de la jupe permettent de définir un exemple d'épaisseur de jupe suffisante pour éviter le gonflement de la tubulure lors d'une augmentation de la pression à l'intérieur de la tubulure. Les relations entre les autres côtes définies dans le tableau ci-dessus permettent la fabrication industrielle du connecteur ainsi que l'assemblage automatique de l'ensemble à usage médical. Par exemple, les côtes D.int1, D.int2 et d.ext1, d.ext2 permettent de définir des appendices et jupes tronconiques. Le fait que la jupe et/ou l'appendice soi(en)t tronconique(s) permet de faciliter l'opération de démoulage du connecteur lorsque celui-ci est fabriqué dans un moule.

Ainsi, l'invention propose un ensemble à usage médical permettant l'administration d'un produit liquide ou visqueux à un patient sans risque de fuite, et ceci même lorsque la pression à l'intérieur de l'ensemble est importante.

Cet ensemble peut être utilisé pour l'administration de différents types de produit quel que soit le type d'injecteur utilisé pour administrer ce produit.

Le lecteur aura compris que de nombreuses modifications peuvent être apportées sans sortir matériellement des nouveaux enseignements et des avantages décrits ici.

## Revendications

1. Ensemble à usage médical pour l'administration d'un produit à un patient, l'ensemble comprenant une tubulure (2) et un connecteur (3) destiné à être raccordé à une extrémité (22) de la tubulure (2), le connecteur (3) comportant un corps (31) et une zone d'interface (33) à l'une des extrémités du corps (31), la zone d'interface (33) comprenant :
- un appendice (331) destiné à être inséré dans la tubulure (2), et
- une jupe (332) entourant l'appendice (331) pour définir une gorge de réception de la tubulure (2) entre l'appendice (331) et la jupe (332), l'appendice et la jupe étant monoblocs et solidaires, les dimensions de l'appendice (331) étant adaptées aux dimensions de la tubulure (2), et la jupe (332) comprenant une face intérieure tronconique en regard de l'appendice (331), le diamètre de la face intérieure de la jupe (332) augmentant en direction de son extrémité libre,
**caractérisé en ce que**
le diamètre externe de l'appendice (331) est supérieur au diamètre intérieur de la tubulure (2) au niveau de son extrémité la plus proche du corps (31) de sorte à exercer un ensemble de forces tendant à dilater la tubulure (2), et **en ce que** le diamètre interne de la jupe (332) est inférieur au diamètre extérieur de la tubulure (2) au niveau de son extrémité la plus proche du corps (31) de sorte à exercer un ensemble de forces tendant à comprimer la tubulure (2).

2. Ensemble selon la revendication précédente, dans lequel le connecteur (3) comprend en outre une tête (321, 322) pour son raccordement à un autre connecteur, et un canal axial (31) sensiblement cylindrique entre l'appendice (331) et la tête (321, 322).

3. Ensemble selon l'une quelconque des revendications précédentes, dans lequel l'appendice (331) comprend une face extérieure tronconique en regard de la jupe, le diamètre de la face extérieure de l'appendice (331) diminuant en direction de son extrémité libre.

4. Ensemble selon l'une quelconque des revendications précédentes, dans lequel le connecteur (3) est moulé en une seule pièce.

5. Ensemble selon l'une quelconque des revendications précédentes, dans lequel l'appendice (331) comprend une section frangible (333) s'étendant selon un axe longitudinal à son extrémité libre, ladite section frangible (333) comportant au moins une ailette (334) s'étendant vers l'extérieur perpendiculairement à l'axe longitudinal, ladite ailette ayant une forme de portion de sphère.

6. Ensemble selon la revendication précédente, dans lequel la section frangible (333) comprend quatre ailettes (334) s'étendant vers l'extérieur perpendiculairement à l'axe longitudinal de la section frangible (333), lesdites ailettes (334) étant de forme en portion de sphère.

7. Ensemble selon l'une quelconque des revendications précédentes, dans lequel le matériau constituant le connecteur (3) est du polycarbonate.

8. Ensemble selon l'une quelconque des revendications précédentes, dans lequel la jupe (332) est constituée dans un matériau transparent ou translucide.

9. Ensemble selon l'une quelconque des revendications précédentes, dans lequel la jupe (332) comprend en outre des cannelures (335) sur sa face extérieure.

10. Ensemble selon la revendication précédente, dans lequel la face extérieure de l'appendice (331) et/ou la face intérieure de la jupe (332) comprend au moins un bourrelet s'étendant vers l'extérieur.

11. Ensemble selon l'une quelconque des revendications précédentes, dans lequel le connecteur (3) comprend en outre un autre appendice destiné à être inséré dans une autre tubulure, et une autre jupe entourant l'autre appendice, les dimensions de l'autre appendice étant prévues de sorte à exercer un ensemble de forces tendant à dilater l'autre tubulure, et les dimensions de l'autre jupe étant prévues de sorte à exercer un ensemble de forces tendant à comprimer l'autre tubulure.

12. Ensemble à usage médical selon l'une des revendications précédentes, lequel comprend en outre une poche souple (1) contenant le produit à administrer, ladite poche étant connectée à l'autre extrémité (21) de la tubulure (2).

13. Utilisation d'un connecteur à usage médical pour raccorder ledit connecteur à une extrémité d'une tubulure, le connecteur comportant un corps (31) et une zone d'interface (33) à l'une des extrémités du corps (31), la zone d'interface (33) comprenant :
- un appendice (331) destiné à être inséré dans la tubulure, et
- une jupe (332) entourant l'appendice de sorte que la jupe et l'appendice définissent une gorge pour la réception de la tubulure entre l'appendice (331) et la jupe (332), l'appendice et la jupe étant monoblocs et solidaires, les dimensions de l'appendice (331) étant adaptées aux dimensions de la tubulure (2), la jupe (332) comprenant une face intérieure tronconique en regard de l'appendice (331), le diamètre de la face intérieure de la jupe (332) augmentant en direction de son extrémité libre,
**caractérisée en ce que**
le diamètre externe de l'appendice (331) est supérieur au diamètre intérieur de la tubulure (2) au niveau de son extrémité la plus proche du corps (31) de sorte à exercer un ensemble de forces tendant à dilater la tubulure (2), et **en ce que** le diamètre interne de la jupe (332) est inférieur au diamètre extérieur de la tubulure (2) au niveau de son extrémité la plus proche du corps (31) de sorte à exercer un ensemble de forces tendant à comprimer la tubulure.

## Patentansprüche

1. Anordnung zur medizinischen Verwendung zur Verabreichung eines Produkts an einen Patienten, wobei die Anordnung einen Schlauch (2) und einen Verbinder (3) umfasst, der dazu bestimmt ist, mit einem Ende (22) des Schlauchs (2) verbunden zu werden, wobei der Verbinder (3) einen Körper (31) und eine Schnittstellenzone (33) mit einem der Enden des Körpers (31) umfasst,
wobei die Schnittstellenzone (33) umfasst:
- einen Ansatz (331), der dazu bestimmt ist, in den Schlauch (2) eingesetzt zu werden, und
- eine Schürze (332), die den Ansatz (331) umgibt, um einen Hals zur Aufnahme des Schlauchs (2) zwischen dem Ansatz (331) und der Schürze (332) zu definieren,
wobei der Ansatz und die Schürze einteilig und fest verbunden sind, wobei die Abmessungen des Ansatzes (331) an die Abmessungen des Schlauchs (2) angepasst sind, und die Schürze (332) eine kegelstumpfförmige Innenfläche gegenüber dem Ansatz (331) umfasst, wobei der Durchmesser der Innenfläche der Schürze (332) in der Richtung seines freien Endes zunimmt,
**dadurch gekennzeichnet, dass**
der Außendurchmesser des Ansatzes (331) größer ist als der Innendurchmesser des Schlauchs (2) auf der Höhe seines dem Körper (31) am nächsten liegenden Endes, um eine Gesamtheit von Kräften auszuüben, die dazu tendieren, den Schlauch (2) zu dilatieren, und dadurch, dass der Innendurchmesser der Schürze (332) kleiner ist als der Außendurchmesser des Schlauchs (2) auf der Höhe seines dem Körper (31) am nächsten liegenden Endes, um eine Gesamtheit von Kräften auszuüben, die dazu tendieren, den Schlauch (2) zu komprimieren.

2. Anordnung nach dem vorhergehenden Anspruch, wobei der Verbinder (3) außerdem einen Kopf (321, 322) für seine Verbindung mit einem anderen Verbinder, und einen im Wesentlichen zylindrischen axialen Kanal (31) zwischen dem Ansatz (331) und dem Kopf (331, 322) umfasst.

3. Anordnung nach einem der vorhergehenden Ansprüche, wobei der Ansatz (331) eine kegelstumpfförmige Außenfläche gegenüber der Schürze umfasst, wobei der Durchmesser der Außenfläche des Ansatzes (331) in der Richtung seines freien Endes abnimmt.

4. Anordnung nach einem der vorhergehenden Ansprüche, wobei der Verbinder (3) in einem einzigen Stück geformt ist.

5. Anordnung nach einem der vorhergehenden Ansprüche, wobei der Ansatz (331) einen zerbrechbaren Abschnitt (333) umfasst, der sich in einer Längsachse zu seinem freien Ende erstreckt, wobei der zerbrechbare Abschnitt (333) mindestens einen Flügel (334) umfasst, der sich nach außen rechtwinklig zur Längsachse erstreckt, wobei der Flügel eine Form eines Kugelabschnitts aufweist.

6. Anordnung nach dem vorhergehenden Anspruch, wobei der zerbrechbare Abschnitt (333) vier Flügel (334) umfasst, die sich nach außen rechtwinklig zur Längsachse des zerbrechbaren Abschnitts (333) erstrecken, wobei die Flügel (334) eine Form eines Kugelabschnitts aufweisen.

7. Anordnung nach einem der vorhergehenden Ansprüche, wobei das Material, das den Verbinder (3) bildet, Polycarbonat ist.

8. Anordnung nach einem der vorhergehenden Ansprüche, wobei die Schürze (332) aus einem transparenten oder lichtdurchlässigen Material besteht.

9. Anordnung nach einem der vorhergehenden Ansprüche, wobei die Schürze (332) außerdem Rillen (335) auf ihrer Außenfläche umfasst.

10. Anordnung nach dem vorhergehenden Anspruch, wobei die Außenfläche des Ansatzes (331) und/oder die Innenfläche der Schürze (332) mindestens einen Wulst umfasst/umfassen, der sich nach außen erstreckt.

11. Anordnung nach einem der vorhergehenden Ansprüche, wobei der Verbinder (3) außerdem einen anderen Ansatz, der dazu bestimmt ist, in einen anderen Schlauch eingesetzt zu werden, und eine andere Schürze, die den anderen Ansatz umgibt, umfasst, wobei die Abmessungen des anderen Ansatzes bereitgestellt sind, um eine Gesamtheit von Kräften auszuüben, die dazu tendieren, den anderen Schlauch zu dilatieren, und wobei die Abmessungen der anderen Schürze bereitgestellt sind, um eine Gesamtheit von Kräften auszuüben, die dazu tendieren, den anderen Schlauch zu komprimieren.

12. Anordnung zur medizinischen Verwendung nach einem der vorhergehenden Ansprüche, welche außerdem einen nachgiebigen Beutel (1) umfasst, der das zu verabreichende Produkt enthält, wobei der Beutel mit dem anderen Ende (21) des Schlauchs (2) verbunden ist.

13. Verwendung eines Verbinders zur medizinischen Verwendung zur Verbindung des Verbinders mit einem Ende eines Schlauchs, wobei der Verbinder einen Körper (31) und eine Schnittstellenzone (33) mit einem der Enden des Körpers (31) umfasst, wobei die Schnittstellenzone (33) umfasst:
- einen Ansatz (331), der dazu bestimmt ist, in den Schlauch eingesetzt zu werden, und
- eine Schürze (332), die den Ansatz umgibt, so dass die Schürze und der Ansatz einen Hals zur Aufnahme des Schlauchs zwischen dem Ansatz (331) und der Schürze (332) definieren,
wobei der Ansatz und die Schürze einteilig und fest verbunden sind, wobei die Abmessungen des Ansatzes (331) an die Abmessungen des Schlauchs (2) angepasst sind, und die Schürze (332) eine kegelstumpfförmige Innenfläche gegenüber dem Ansatz (331) umfasst, wobei der Durchmesser der Innenfläche der Schürze (332) in der Richtung seines freien Endes zunimmt,
**dadurch gekennzeichnet, dass**
der Außendurchmesser des Ansatzes (331) größer ist als der Innendurchmesser des Schlauchs (2) auf der Höhe seines dem Körper (31) am nächsten liegenden Endes, um eine Gesamtheit von Kräften auszuüben, die dazu tendieren, den Schlauch (2) zu dilatieren, und dadurch, dass der Innendurchmesser der Schürze (332) kleiner ist als der Außendurchmesser des Schlauchs (2) auf der Höhe seines dem Körper (31) am nächsten liegenden Endes, um eine Gesamtheit von Kräften auszuüben, die dazu tendieren, den Schlauch zu komprimieren.

## Claims

1. Assembly for medical use for administering a product to a patient, the assembly comprising a tube (2) and a connector (3) designed to be attached to an end (22) of the tube (2), the connector (3) having a body (31) and an interface zone (33) at one of the ends of the body (31),
the interface zone (33) comprising:
- an appendage (331) designed to be inserted into the tube (2), and
- a skirt (332) surrounding the appendage (331) in order to define a groove for receiving the tube (2) between the appendage (331) and the skirt (332),
the appendage and the skirt being in one piece and integrally connected,
the dimensions of the appendage (331) being adapted to the dimensions of the tube (2), and the skirt (332) comprising a frustoconical inner face opposite the appendage (331), the diameter of the inner face of the skirt (332) increasing in the direction of its free end,
**characterized in that**
the external diameter of the appendage (331) is greater than the internal diameter of the tube (2) at its end nearest the body (31), in such a way as to exert a set of forces tending to dilate the tube (2), and **in that** the internal diameter of the skirt (332) is less than the external diameter of the tube (2) at its end nearest the body (31), in such a way as to exert a set of forces tending to compress the tube (2) .

2. Assembly according to the preceding claim, in which the connector (3) additionally comprises a head (321, 322) for attaching it to another connector, and a substantially cylindrical axial channel (31) between the appendage (331) and the head (321, 322).

3. Assembly according to either of the preceding claims, in which the appendage (331) comprises a frustoconical outer face opposite the skirt, the diameter of the outer face of the appendage (331) decreasing in the direction of its free end.

4. Assembly according to any of the preceding claims, in which the connector (3) is moulded in one piece.

5. Assembly according to any of the preceding claims, in which the appendage (331) comprises a breakable portion (333) extending along a longitudinal axis at its free end, said breakable portion (333) having at least one wing (334) extending outwardly perpendicular to the longitudinal axis, said wing having the shape of a portion of a sphere.

6. Assembly according to the preceding claim, in which the breakable portion (333) comprises four wings (334) extending outwardly perpendicular to the longitudinal axis of the breakable portion (333), said wings (334) having the shape of a portion of a sphere.

7. Assembly according to any of the preceding claims, in which the material from which the connector (3) is made is polycarbonate.

8. Assembly according to any of the preceding claims, in which the skirt (332) is made of a transparent or translucent material.

9. Assembly according to any of the preceding claims, in which the skirt (332) additionally comprises ribs (335) on its outer face.

10. Assembly according to the preceding claim, in which the outer face of the appendage (331) and/or the inner face of the skirt (332) comprises at least one bead extending toward the outside.

11. Assembly according to any of the preceding claims, in which the connector (3) additionally comprises another appendage designed to be inserted into another tube, and another skirt surrounding the other appendage, the dimensions of the other appendage being provided to exert a set of forces tending to dilate the other tube, and the dimensions of the other skirt being provided to exert a set of forces tending to compress the other tube.

12. Assembly for medical use according to one of the preceding claims, which assembly additionally comprises a flexible bag (1) containing the product to be administered, said bag being connected to the other end (21) of the tube (2).

13. Use of a connector for medical use for attaching said connector to an end of a tube, the connector having a body (31) and an interface zone (33) at one of the ends of the body (31), the interface zone (33) comprising:
- an appendage (331) designed to be inserted into the tube, and
- a skirt (332) surrounding the appendage so that the skirt and the appendage define a groove for receiving the tube between the appendage (331) and the skirt (332),
the appendage and the skirt being in one piece and integrally connected, the dimensions of the appendage (331) being adapted to the dimensions of the tube (2), the skirt (332) comprising a frustoconical inner face opposite the appendage (331), the diameter of the inner face of the skirt (332) increasing in the direction of its free end,
**characterized in that**
the external diameter of the appendage (331) is greater than the internal diameter of the tube (2) at its end nearest the body (31), in such a way as to exert a set of forces tending to dilate the tube (2), and **in that** the internal diameter of the skirt (332) is less than the external diameter of the tube (2) at its end nearest the body (31), in such a way as to exert a set of forces tending to compress the tube (2).
